# EUROPEAN PATENT SPECIFICATION

(11) **EP 1 829 565 B1**
(45) Date of publication and mention of the grant of the patent: **17.02.2010**
(21) Application number: 07012288.2
(22) Date of filing: 20.12.2002
(51) Int. Cl.: A61L 24/02, A61L 27/12

(54) **A new bone mineral substitute**
Neuer Knochenmineralienersatzstoff
Nouveau substitut mineral d'os

(30) Priority: 20.12.2001 SE 0104359; 20.12.2001 US 341282 P
(43) Date of publication of application: 05.09.2007
(62) Divisional of application: 02793727.5
(73) Proprietor: Bone Support AB, 223 70 Lund (SE)
(72) Inventor: Lidgren, Lars Ake Alvar, 22 221 Lund (SE)
(74) Representative: Nilausen, Kim

(56) References cited:
- EP-A1- 0 639 382
- EP-A2- 0 520 690
- WO-A-02/05861
- WO-A-91/00252
- WO-A-91/17722
- WO-A-99/62570

## Description

The invention refers to an artificial bone mineral substitute material as well as a composition for the same, which have improved radio-opacity. The invention also refers to the use of composition for an artificial bone mineral substitute material as an X-ray contrast medium.

The life expectancy of the world population has increased tremendously during the last 50 years. Our population is living longer than ever. The next ten years, it has been forecasted that there will be more people over 60 years of age than less than twenty years of age in Europe. More people will need medical help for diseases related to age, which will increase the pressure of the hospitals.

Bone is the second most common material to be transplanted after blood. The most reliable method to repair bone defects is to use autogenous bone, i.e. bone taken from another site in the body. Autografts are osteogenic, i.e. derived from or composed of any tissue which is concerned with the growth or repair of bone. However, problems may occur at the second surgical site where the graft is taken. To avoid this extra trauma allografts can be used, i.e. bone graft between individuals of the same species. Allografts have a lower osteogenic capacity than autografts and the rate of new bone formation might be lower. They also have a higher resorption rate, a larger immunogenic response and less revascularisation of the recipient. Allografts must also be controlled for viruses since they can transfer, for example, HIV and hepatitis. The use of allografts is now the most common method for bone transplantation and repairing of bone defects.

The problems with using autografts and the limited supply of bones at most hospitals has led to a lot of research in materials that can be used as bone substitutes. The ideal bone substitute should be biocompatible, injectable through a needle, self-setting, osteoconductive, resorbable and replaced by new normal bone.

An injectable bone graft substitute can improve the treatment of osteoporosis. Osteoporosis is a disease that acts upon older people. Bone is live tissue, new cells are formed and some cells die daily. In a year 10-30 % of our entire skeleton is remodeled. Osteoporosis leads to a total skeleton mass reduction because of a non-equilibrium state between cells that form bone (osteoblasts) and cells that resorb bone (osteoclasts). When the resorption rate of bone is higher than the rate of bone formation it leads to a total reduction of bone. The skeleton becomes weaker and finally a fracture occurs either by reason of a fall or just because the skeleton can not withstand the weight of the body. When the spine becomes weaker it will be curved and compressed.

There are two types of bone: trabecular and cortical. The outside layer of bone usually consists of cortical bone, which is very dense and solid, trabecular bone is much more porous and is found inside of the bones. Osteoporosis initially effects the trabecular bone.

Fractures resulting from osteoporosis are very troublesome to treat. The skeleton is fragile and difficult to stabilise with screws and plates. Even if the screw insertion succeeds it often becomes loose when the patient later starts to move. To attach the screws to the bone a bone graft substitute can be injected in the drilled holes before screw insertion. The result is that the screw fixation improves and the patient recovers faster with less pain. If a thin layer of bone substitute is used it can be difficult to see in an X-ray picture. To improve the visibility, it could be possible to add a radiographic contrast medium to the material. To stabilise the spine, a bone graft substitute can be injected. If a bone substitute leaks from the spine, it will cause pressure on nearby tissues and nerves which will cause pain and eventually nerve injury.

One of the materials that has been used for stabilising the spine is the bone cement polymethylmetacrylate (PMMA). PMMA is a chain polymer that is glassy at room temperature. PMMA is a good material for intraocular lenses and hard contact lenses. It is also used to fixate the metal implant in hip joints and for skull defect reconstruction and vertebroplasty. PMMA is not resorbed in the bone. PMMA has a couple of drawbacks. For example PMMA cures with a strongly exothermic reaction, potentially damaging adjacent soft tissue structures i.e. cell death, particularly in the event of cement extrusion. Therefore, PMMA is not an optimal material for this indication.

Presently, mainly three kinds of bone substitutes are used for repairing bone: calcium phosphate, hydroxyapatite and calcium sulfate.

Calcium phosphates are suitable as bone substitutes because of their bioactive properties, i.e. having an effect on or obtaining response from living tissue. They have low fatigue properties relative to bone and can only be used in non-weight bearing areas. Their resorption rate is relatively slow, at least six months.

There are two different categories of calcium phosphates: CaP obtained by precipitation from an aqueous solution at room temperature (low-temperature CaP) and CaP obtained by a thermal reaction (high-temperature CaP). The calcium phosphates used in medicine are high-temperature CaP, for example α-tricalcium phosphate (α-TCP) and hydroxypatite (HA). Low-temperature CaP is used to synthesize high-temperature CaP.

Tricalcium phosphate (Ca₃(PO₄)₂) exists in two forms: α-TCP and β-TCP. The crystal structure of α-TCP is monoclinic and consists of columns of cations while the β-TCP has a rhombohedral structure. β-TCP is stable up to 1180 °C and α-TCP is stable between 1180-1470°C. α-TCP forms by heating β-TCP above 1180 °C and quenching it to retain its structure. α-TCP is less stable than β-TCP and forms the stiffer material calcium-deficient HA when mixed with water, see formula 1.

3 Ca₃(PO₄)₂ + H₂O → Ca₉(HPO₄)(PO₄)₅OH (1)

Hydroxyapatite is the most stable calcium phosphate and the primary non-organic component of bone. Most of the bone graft substitutes on the market are made of hydroxyapatite. HA is highly crystalline and the least soluble of the calcium phosphates.

Hydroxyapatite and tri-calcium phosphate are the most common calcium phosphates used to fill bone defects and as implant coatings. They have low fatigue properties relative bone and can only be used in non-weight bearing areas. Their resorption rate is relatively slow, from six months to several years. It is possible to increase the rate of degradation slightly by increasing the surface area of the material, decreasing the crystallinity and the crystal perfection and decreasing the size of crystals and grains in the material. A higher resorption rate can be preferable to encourage bone formation.

Hydroxyapatite Ca₁₀(PO₄)₆(OH)₂) is the major mineral component of bone. Artificial hydroxyapatite (HA) has a chemical and crystallographic structure similar to the natural HA of bone and has been studied as a bone replacement material for over 30 years. HA is highly crystalline and the most stable CaP in an aqueous solution. HA is the least soluble calcium phosphate at pH over 4.2, which means that other calcium phosphates with a pH over 4.2 will tend to dissolve and form precipitated HA. HA is biocompatible, not osteogenic but has osteoconductive properties. Most of the bone substitutes on the market are made of HA. They are regarded as degradable, but their degradation time is very long. The final compressive strength depends on which type of HA is used. If spray-dried HA is used the compressive strength is very low. Even the shape and size of the powder affect the mechanical strength.

There are several forms of HA that have been used experimentally and clinically: solid ceramic blocks, porous blocks and solid and porous particulate. The major use of HA is in the oral surgery, where the particulate HA is used. Particulate HA is inconvenient to use for orthopaedic applications because particles often migrate from the implant site before bone ingrowth secure them in place. Porous HA blocks have successfully been used for craniofacial reconstructions and orthopaedic applications, but they are difficult to shape.

Research in calcium sulfates as a bone graft substitute has been carried out for more than a century. Several investigators have reported experiments using calcium sulfate hemihydrate, commonly known as Plaster of Paris (PoP). This material is well accepted by the body, the resorption rate is faster than the rate of bone ingrowth and the mechanical strength for Plaster of Paris is low.

Calcium sulfate hemihydrate exists in two forms, α-form and β-form. The α-form has monoclinic structure and consists of compact, well-formed, and transparent large primary particles. The β-form has rhombohedral structure and consists of rugged secondary particles made up of extremely small crystals.

Plaster of Paris is made from calcium sulfate dihydrate (gypsum) through dehydration, see reaction 1. The gypsum is ground and heated until about 75 % of the water is gone and CaSO₄·½H₂O is obtained. When Plaster of Paris is mixed with water, an exothermic reaction takes place with gypsum as product, which is called rehydration of gypsum, see reaction 1. The structure of gypsum consists of layers of alternate SO₄²⁻ ions strongly bonded to Ca²⁺ and sheets of water molecules. 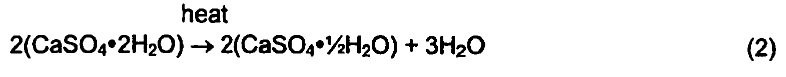 2(CaSO₄•½H₂O) + 3H₂O → 2(CaSO₄•2H₂O) + heat (3)

If calcium sulfate dihydrate is added to calcium sulfate hemihydrate the setting time will decrease because the nucleation time is eliminated. Crystal growing can start directly on the calcium sulfate dihydrate particles which thus act as an accelerator.

Disodium hydrogen phosphate (Na₂HPO₄) is used as an accelerator for the calcium phosphate compositions. When Na₂HPO₄ is added to the cement powder, the (PO4)³⁻ ions will bond to Ca²⁺ ions and the precipitation rate will increase which gives a shorter setting time.

Research in the use of different radiocontrast materials in bone cement, i.e. PMMA, has been conducted and the influence of radiocontrast media in PMMA has been studied. The radiocontrast agents, bariumsulfate (BaSO₄) and zirconium dioxide (ZrO₂), are commonly added to polymethylmethacrylite (PMMA) to ensure X-ray visibility and to ease radiological assessments. These two materials have negative side effects in that they may cause pathological bone resorption as well as cause damage to the articulating surface if they enter the joint space, a marked increase in the production of polyethylene wear debris being obtained. Furthermore, if included in bone substitutes, such an X-ray dense bone substitute may leak from the spine and cause nerve damages.

When such a ceramic bone is resorbed with time, it will subsequently contact other tissues. Contrast agents in the form of metals, zirconium oxide, or barium sulfate will remain at the site of treatment or will be spread as small particles to other organs, such as the lungs, and/or will ultimately be transported to the kidneys where they will be trapped. Thus, it is important that the contrast agent dissolves in the blood and is disposed off *via* the kidneys as primary urine.

Water soluble non-ionic X-ray contrast agents have been used in connection with polymeric materials. In WO 99/62570 such agents have been used for preventing the release of particles from the bone cement, i.e. the plastic material, which contribute to the wear of adjacent surfaces at the surgical site.

The object of this invention is to provide an artificial bone mineral substitute material, whereby the above-mentioned problems are eliminated or reduced.

Another object of the invention is to provide an artificial bone mineral substitute material which exhibits positive radiophysical effects in comparison with the state of the art.

Still another object is to provide a bone graft substitute which improves the possibility to detect leakage during the operation.

A further object of the invention is to provide a composition which after hardening results in a "safe" ceramic implant comprising a biocompatible X-ray contrast medium, whereby a leakage of the contrast agent from the site of injection is allowed, which does not give rise to inflammation in the vicinity of the site of injection and is thus atoxic for e.g. the nervous system.

Yet a further object is to provide a composition which does not exhibit the drawbacks of high viscosity at delivery and which provides improved rheological properties and allows injection at distant sites in comparison with the state of the art.

Still a further object of the invention is to provide a composition, whereby ceramic materials do not have to be included in the X-ray contrast medium.

Still another object of the invention is to provide a composition, whereby it is possible to follow the healing of bone defects and bone fractures.

Still yet a further object of the invention is to provide a composition which can be used in applications of bone defects communicating with joints.

These objects as well as other objects that will be apparent from the following description are accomplished by the artificial bone mineral substitute material as well as the composition for the same as claimed.

In the drawings
FIG 1 shows the relation between injection time and amount of iohexol for a constant amount of accelerator for a calcium sulfate based composition;
FIG 2 shows the relation between injection time and amount of iohexol for calcium phosphate based compounds; and
FIG 3 shows the relation between setting time and amount of iohexol for a calcium phosphate compound with a liquid/powder ratio of 0.28.

The inventive composition as well as the inventive artificial bone mineral substitute material comprises at least one ceramic material and at least one water soluble non-ionic X-ray contrast agent. It is preferred that the composition is injectable and that the artificial bone obtained is bioresorbable.

Suitable conventional water soluble non-ionic X-ray contrast agents are iodinated aromatic compounds, which have one or several aromatic nuclei that are at least triiodo-substituted. Such agents are shown in US 5,695,742 and comprise CAS (Chemical Abstract Service) registration numbers 31112-62-6 (metrizamide), 60166-93-0 (iopamidol), 78649-41-9 (iomeprol), 73334-07-3 (iopromide), 877771-40-2 (ioversol), 66108-95-0 (iohexol), 89797-00-2 (iopentol), 107793-72-6 (ioxilan), 99139-49-8 (II-1), 75751-89-2 (iogulamide), 63941-73-1 (loglucol), 63941-74-2 (ioglucamide), 56562-79-9 (ioglunide), 76984-84-0 (MP-7011), 64965-50-0 (MP-7012), 77111-65-0 (MP-10007), 79944-49-3 (VA-7-88), 79944-51-7 (also shown in EP 033426), 79211-10-2 (iosimide), 79211-34-0 (iocibidol), 103876-29-5 (also shown in EP 0177414), 141660-63-1 (iofratol), 92339-11-2 (iodixanol), 79770-24-4 (iotrol), 71767-13-0 (iotasul), 81045-33-2 (iodecol), 143200-04-8 (also shown in WO 92/086), 143199-77-3 (also shown in WO 92/08691), 143200-00-4 (also shown in WO 92/08691), 78341-84-1 (also shown in US 4348377), 122731-47-9 (also shown in EP 0308364), 122731-49-1 (also shown in EP 0308364), 99139-65-8 (also shown in WO 85/01727), 99139-62-5 (also shown in WO 85/01727), and 78341-84-1 (also shown in EP 0023992).

Other such water soluble non-ionic X-ray contrast agents are shown in US 5,447,711 and comprise iotrolan, ioxaglate, iodecimol, and iosarcol. Other suitable contrast agents are iotusal, ioxilane, and iofrotal.

Preferably, the water soluble non-ionic X-ray contrast agent has a low osmomolality such as iohexol, iodixanol, ioversol, iopamidol, and iotrolane.

For example, iohexol (C₁₉H₂₆IN₃O₉) or its dimer iodixanol can with advantage be used as a water soluble non-ionic X-ray contrast agent. These substances do not influence bone formation and they have a good biocompability in bone. They are used for different purposes in medicine. For example it can be used for patients with kidney failure to determine the rate of plasma clearance by the kidney.

Thus, the inventive composition as well as the inventive artificial bone mineral substitute material comprises at least one water soluble non-ionic X-ray contrast agent, preferably iohexol or its dimer iodixanol. When used in the inventive composition, the water soluble non-ionic X-ray contrast agent should have a concentration between 2 and 20 weight% of the total weight of the powder components in the same.

Of course, other contrast agents can also be included in the inventive artificial bone mineral substitute material as well as the composition for the same.

Suitable ceramics are calcium sulfates and calcium phosphates.

Examples of calcium phosphate ceramics are a-tricalcium phosphate, hydroxyapatite, dicalcium phosphate dihydrate, anhydrous dicalcium phosphate, tetracalcium phosphate, β-tricalcium phosphate, calcium-deficient hydroxyapatite, monocalcium phosphate monohydrate, monocalcium phosphate, calcium pyrophosphate, precipitated hydroxyapatite, carbonated apatite (dahlite), octocalcium phosphate, amorphous calcium phosphate, oxyapatite, and carbonatoapatite.

Suitable types of calcium phosphates are shown in Table 1 below.

| **Ca/P** | *Calcium phosphate* | **Formula** |
|---|---|---|
| 1.35 | Amorphous calcium phosphate I ACP | - |
| 1.35 | Amorphous calcium phosphate II ACP | - |
| 0.5 | Monocalcium phosphate monohydrate MCPM | Ca(H₂PO₄)₂•2H₂O |
| 1.0 | Dicalcium phosphate dihydrate DCPD (brushite) | CaHPO₄•2H₂O |
| 1.33 | Octacalcium phosphate | Ca₈(HPO₄)₂(PO₄)₄•5H₂O |
| 1.5 | Calcium deficient hydroxyapatite (CDHA) | Ca₉(HPO₄)(PO₄)₅(OH) |
| 1.5 | Tricalcium phosphate (TCP) | Ca₃(PO₄)₂ |
| 1.67 | Hydoxyapatite (HA) | Ca₁₀(PO₄)₆(OH)₂ |

Preferably, the calcium phosphate has a Ca/P-ratio between 0.5 and 2. Likewise, it is preferred that the particulate calcium phosphate is hydroxyapatite (HA), tri-calcium-phosphate (TCP), or a mixture thereof.

In the composition the particulate calcium phosphate should have a particle size of less than 20 µm, preferably less than 10 µm.

The calcium sulfate can be calcium sulfate a-hemihydrate, calcium sulfate β-hemihydrate, or calcium sulfate β-dihydrate.

Calcium carbonate can also be used as a ceramic.

The inventive composition for an artificial bone mineral substitute material can in itself be used as an X-ray contrast medium. An additive X-ray effect is obtained by means of the composition according to the invention, since the X-ray contrast ability of its ceramic component is utilized. The inclusion of at least one water soluble non-ionic X-ray contrast agent in the composition increases the original X-ray density of the X-ray dense bone substitute. Thus, no further ceramic radio contrast agents, such as bariumsulfate and zirconium dioxide, have to be included in the composition for an artificial bone mineral substitute material according to the invention. Such hard ceramic particles will wear joints when torn off from the bone substitute. In the joints they will cause physical damage and eventually result in inflammatory reactions.

Thus, the inventive composition, which comprises at least one water soluble non-ionic X-ray contrast agent, can be injected adjacent to joints, and the resulting artificial bone mineral substitute material can be used for bone defects communicating with joints. Such applications include the repair of osteochondral joints defects as well as fractures or bone defects involving a joint.

In addition, the dual origin of X-ray density can be further exploited, for example in order to follow the healing process after implantation of the inventive composition in a human or animal body. When the obtained artificial bone mineral substitute material, which comprises at least one water soluble non-ionic X-ray contrast agent, is replaced by ingrowing bone, the water soluble agent will slowly disappear. This results in a progressive decline in X-ray density, which can be monitored.

In order to increase the healing process the inventive composition should also comprise at least one osteogenic factor. In this connection an osteogenic factor is a substance that influences bone turnover, either by increasing bone formation or by decreasing bone breakdown.

Suitable bone inducing (stimulating and/or accelerating) substances are growth factors and hormones. Growth factors and derivatives thereof are preferred, which are locally acting, e.g. the BMP family (Bone Morphogenetic Proteins). It is also preferred to use autologous growth factors in order to accelerate bone growth.

Examples of such bone stimulating compounds are parathyorid hormones and derivatives thereof, estrogenes, progesterones, androgenes, testosterones, calcitonin, somatomedin, and oxytocin.

The enamel matrix proteins amelin-1, amelin-2 and ameloblastin as well as the cholesterol-lowering compound statin can also be included in order to induce, stimulate and/or accelerate bone formation.

Examples of suitable bone breakdown inhibitors are biphosphonates, osteocalcin, osteonectin and derivates thereof, which can be included in the inventive composition and the resulting material.

The inventive composition may also include at least one accelerator, which preferably is calcium sulfate dihydrate and/or disodium hydrogen phosphate. When calcium sulfate dihydrate is used, it should have a concentration between 0.1 and 10 weight% of the total weight of the powder components in the composition.

A preferred injectable composition comprises particulate calcium sulfate hemihydrate, particulate calcium phosphate. The composition may also include particulate calcium sulfate dihydrate as an accelerator and optionally vitamin E. When vitamin E is included, it should have a concentration between 0.1 and 10 weight% of the total weight of the powder components in the composition.

Likewise, the inventive composition as well as the resulting artificial bone mineral substitute material may further include kalciferols, kalcitriols, as well as other D vitamins and derivates thereof. These compounds help to regulate calcium metabolism and normal calcification of the bones in the body as well as influence the utilization of mineral phosphorus.

Compounds with static or cidal effects against invading foreign living material can also be included. Such compounds include natural antiobiotics as well as other semisynthetic and synthetic antibacterial compounds, antiviral compounds, antifungal compounds, and antiparasite compounds.

Cytostatics and other chemotherapy substances can also be included in the composition as well as the material according to the invention.

When at least one powdered ceramic is a calcium sulfate hemihydrate and at least one of hydroxyapatite and β-tricalcium phosphate, the at least one of hydroxyapatite and β-tricalcium phosphate has a concentration between 20 and 60 weight% of the total weight of the powder components in the inventive composition.

When at least one powdered ceramic is a calcium sulfate hemihydrate and α-tricalcium phosphate, the calcium sulfate hemihydrate has a concentration between 1 and 30 weight% and the α-tricalcium phosphate has a concentration between 50 and 99 weight% of the total weight of the powder components in the inventive composition, respectively

Other injectable compositions for an artificial bone mineral substitute material are similarly based on tetracalciumphosphate (TTCP) and hydroxyapatite (HA); α-tricalcium phosphate (TCP) and tetracalciumphosphate; α-tricalcium phosphate, tetracalciumphosphate, and citric acid; α-tricalcium phosphate, tetracalciumphosphate, dicalcium sulfate dihydrate (DCPD), and hydroxyapatite; α-tricalcium phosphate, dicalcium phosphate (DCP), calcium carbonate, and hydroxyapatite; and hydroxyapatite acrylic monomers.

By including a water-soluble non-ionic X-ray contrast agent the strength of the bone mineral substitute is somewhat decreased. This can, however, be compensated by reducing the water content thereof. Nevertheless, the inventive artificial bone mineral substitute material is preferably used in connection with indications, in which a high strength is not required but when a high X-ray contrast is required.

An additional advantage of the artificial bone mineral substitute material according to the invention is that the water soluble non-ionic X-ray contrast agent also functions effectively in its solid dry state. Thus, the water-soluble non-ionic X-ray contrast agent is mixed into the inventive composition as well as the inventive artificial bone mineral substitute material and is evenly distributed in the same.

Preferably, the water soluble non-ionic X-ray contrast agent is mixed into the dry sulfate component as a dry powder, or it is dissolved in the aqueous liquid of the composition. It is also preferred that the aqueous liquid component is distilled water.

In the composition according to the invention a ratio between the powder components and the aqueous liquid component, i.e. the liquid/powder ratio, should be between 0.1 and 0.4 ml/g.

In a preferred embodiment of the inventive injectable composition, which comprises calcium sulfate hemihydrate, calcium phosphate, and an aqueous liquid, the water is allowed to react with the dry sulfate or phosphate components of the composition. This results in a crystallization of the ceramic component(s).

When the artificial bone mineral substitute material is resorbed in the body, the water in the blood will ultimately dissolve the sulfate ceramic and the water soluble non-ionic X-ray contrast agent will be released and disposed of.

Likewise, in a mixture of sulfate/phosphate or sulfate/hydroxyapatite, the water soluble non-ionic X-ray contrast agent will be dissolved in a more limited extent since it will be locked within the bone mineral substitute material.

For example, a composite of hydroxyapatite and calcium sulfate results in a material with much better handling properties than hydroxyapatite alone. The mixture can be injected or manually inserted to a bone defect under pressure. The hydroxyapatite particles are held in place by the Plaster of Paris which initially acts as a binder. When the Plaster of Paris resorbs, a matrix with controlled porosity is obtained, which supports bone ingrowth. If the amount of hydroxyapatite is at least 40 %, the temperature of the setting reaction is decreased which is another reason for adding hydroxyapatite to Plaster of Paris.

Thus, by mixing the water soluble non-ionic X-ray contrast agent as a powder in the dry sulfate or phosphate component, respectively, the dissolution of the same with time can be controlled.

The very viscous bone substrate is more easily injected than those according to the state of the art, i.e. positive rheological properties are obtained when a water soluble non-ionic X-ray contrast agent is included in the composition for a bone mineral substitute material.

### EXAMPLES

The invention will now be further described and illustrated by reference to the following examples. It should be noted, however, that these examples should not be construed as limiting the invention in any way.

### Example 1. X-ray analysis.

The X-ray visibility was determined with three different amounts of iohexol in both calcium phosphate and calcium sulfate based compositions. Two different tests were performed. In one test the samples used for setting tests were subjected to X-rays and the visibility of the three samples were compared. Four samples of each material were studied. The liquid/powder ratio was the same for the three tests. In the other test, holes were drilled in bovine vertebrae. The holes were 10 mm in diameter and the depth was 10 mm. The holes were filled with material by injection through a syringe and analysed by X-ray. The visibility of the different materials was compared with the visibility of bone. The X-ray settings were 60kV and 40mAs. The same equipment was used for both tests. Table 2 shows the compositions based on calcium sulfate hemihydrate, and in Table 3 the compositions based on calcium phosphate are shown. An aluminium ladder scaled from 1-5 was used for the comparison, 5 being the lowest visibility and 1 the highest.

**Table 2**

| lohexol (wt%) | PoP (wt%) | HA (wt%) | Accelerator (wt%) | Liquid/powder ratio |
|---|---|---|---|---|
| 10 | 53.28 | 36 | 0.72 | 0.21 |
| 5 | 56.24 | 38 | 0.76 | 0.21 |
| 0 | 59.2 | 40 | 0.8 | 0.21 |

**Table 3**

| lohexol (wt%) | TCP (wt%) | PoP (wt%) | Calcium sulfate dihydrate (wt%) | Liquid/powder ratio |
|---|---|---|---|---|
| 10 | 70 | 19.8 | 0.2 | 0.28 |
| 5 | 75 | 19.8 | 0.2 | 0.28 |
| 0 | 80 | 19.8 | 0.2 | 0.28 |

It was found that the X-ray visibility was improved by adding a radio-contrast medium such as iohexol or its dimer iodixanol. A concentration of 10 % iohexol makes the visibility significantly better both in calcium phosphates and calcium sulfates compared to bone. There is no differ ence between the visibility in calcium phosphates and calcium sulfates.

### Example 2. Test of Injectability.

An Instron 8511.20 equipment was used. Twenty gram of each powder was mixed with distilled water and added to a 10 ml syringe with a 2 mm diameter opening. The syringe was placed in the Instron machine and compressed at a constant rate of 10 mm/min. For injection of the paste by hand, the maximum force corresponds to about 120 N. The Injection time was calculated as the time from start mixing powder and liquid until the force is more than 120 N. The final value for the injection time was taken as a mean value of six tests. For clinical applications, the required injection time is 3 to 8 minutes.

The use of the inventive composition for an artificial bone mineral substitute material as an injectable X-ray contrast medium was tested. Three samples of a contrast medium with or without a water soluble non-ionic X-ray contrast agent were tested, the total injection time as well as the weight extruded from syringe being measured. The results are shown in Table 4 below.

**Table 4**

| Contrast medium | Total injection time (min) | Weight extruded from syringe (%) |
|---|---|---|
| Water (deionized) L/P=0.28 | | |
| Sample 1 | 4.23 | 22 |
| 2 | 4.17 | 20 |
| 3 | 4.28 | 16 |
| lohexol 10.0% L/P=0.326 H₂O/P=0.28 | | |
| Sample 1 | 6.28 | 77 |
| 2 | 6.77 | 68 |
| 3 | 6.78 | 69 |

The utility, i.e. the injectability, of the contrast medium is markedly increased after the inclusion of the water soluble non-ionic X-ray contrast agent.

Further material compositions tested for injection time are shown in Table 5 and 6. In Table 5 the compositions based on calcium sulfate hemihydrate are shown, which were tested for in injection time tests. Table 6 shows the corresponding compositions based on calcium phosphate.

**Table 5**

| lohexol (wt%) | PoP (wt%) | HA (wt%) | Accel. (wt%) | Liquid/powder ratio |
|---|---|---|---|---|
| 10 | 53.64 | 36 | 0.36 | 0.21 |
| 10 | 53.28 | 36 | 0.72 | 0.21 |
| 10 | 52.92 | 36 | 1.08 | 0.21 |
| 5 | 56.62 | 38 | 0.38 | 0.21 |
| 0 | 59.6 | 40 | 0.4 | 0.21 |

**Table 6.**

| lohexol (wt%) | α-TCP (wt%) | PoP (wt%) | Calcium sulfate dihydrate (wt%) | Liquid/powder ratio |
|---|---|---|---|---|
| 10 | 70 | 19.8 | 0.2 | 0.26 |
| 5 | 75 | 19.8 | 0.2 | 0.26 |
| 0 | 80 | 19.8 | 0.2 | 0.26 |

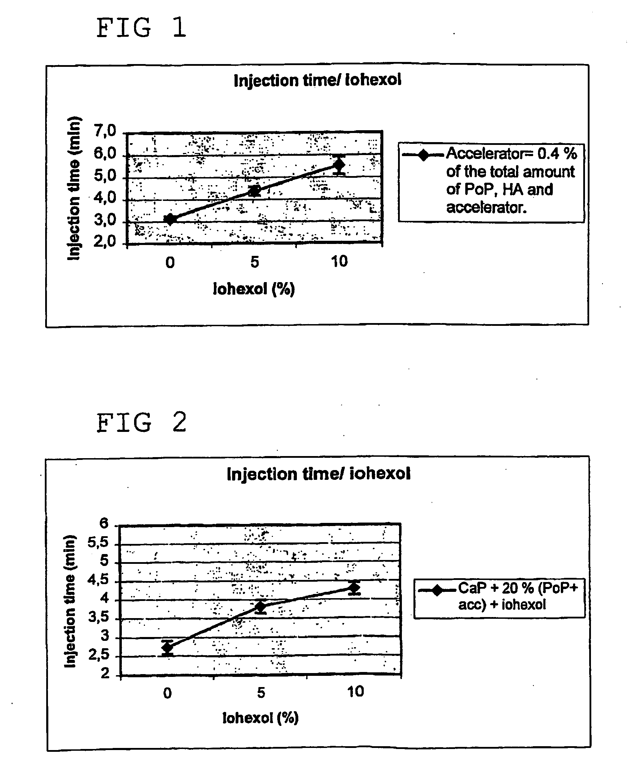

With calcium sulfate hemihydrate as a ceramic the injection time was found to be dependent on the amount of iohexol or its dimer iodixanol. The influence of iohexol was investigated in the same way, 0, 5 and 10 % of iohexol were tested with a constant amount of accelerator. The tests showed that an increase in amount of iohexol increases the injection time, see FIG 1.

In addition, lohexol increases the injection time when calcium phosophate is used as a ceramic, see FIG 2.

### Example 3. Test of Setting.

To determine the time of setting the ASTM standard test method by Gillmore needles has been used. The Gillmore apparatus consists of two needles with different weights applied. The initial needle has a diameter of 2.12 ± 0.05 mm and a weight of 113 ± 0.5g is applied. The final needle has a diameter of 1.06 ± 0.05 mm and a weight of 453.6 ± 0.5g.

To prepare test specimens the iohexol is first dissolved in distilled water and then added to the powder mixture. 10 gram of powder, with the iohexol included was used in each test. After mixing the paste is put into two moulds to form the test specimens. The initial and final setting time was taken as a mean value of the two tests. This procedure was repeated six times. The result presented is the mean value of the six tests.

The initial setting time is the time from the moment water is added to the bone substitute powder until the initial needle leaves no mark on the surface of the specimen. The final setting time is the time required for the paste to set so much that the final needle leaves no mark on the surface.

The ultimate amount of HA is 40 wt% if a mixture of PoP, HA and accelerator is used. Therefore the amount of HA is related to PoP and the accelerator. When materials with 0, 5, 10 wt% of iohexol were tested the iohexol was first calculated and then 40 % HA and for example 0.4 wt% accelerator and 59.6 wt% PoP of the rest were used. The compositions of the tested materials are shown in Table 7.

**Table 7**

| lohexol (wt%) | PoP (wt%) | HA (wt%) | Accel. (wt%) | Liquid/powder ratio |
|---|---|---|---|---|
| 10 | 53.64 | 36 | 0.36 | 0.21 /0.19/0.17 |
| 10 | 53.28 | 36 | 0.72 | 0.21 |
| 10 | 52.92 | 36 | 1.08 | 0.21 |
| 5 | 56.62 | 38 | 0.38 | 0.21 |
| 5 | 56.24 | 38 | 0.76 | 0.21 |
| 5 | 55.86 | 38 | 1.14 | 0.21 |
| 0 | 59.6 | 40 | 0.4 | 0.21 |
| 0 | 59.2 | 40 | 0.8 | 0.21 |
| 0 | 58.8 | 40 | 1.2 | 0.21 |

The final setting time for calcium sulfate hemihydrate and calcium phosphate based bone substitutes should preferably be less than 15 minutes.

It was found that the setting time is dependent on amount iohexol; an adding iohexol to calcium sulfate hemihydrate compositions increases the setting time. Furthermore, the setting time decreases with increased amount of accelerator.

The results of the setting test with different amounts of accelerator and iohexol as in Table 7 with a liquid/powder ratio of 0.21 are shown in Table 8 below.

**Table 8**

| lohexol (wt%) | Accel. (wt%) | Initial setting time (min) | Final setting time (min) |
|---|---|---|---|
| 10 | 0.36 | 6.8 ± 0.5 | 13.4 ± 0.6 |
| 10 | 0.72 | 5.8 ± 0.2 | 12.9 ± 0.4 |
| 10 | 1.08 | 5.5 ± 0.3 | 12.7 ± 0.5 |
| 5 | 0.38 | 5.4 ± 0.2 | 11.2 ± 0.4 |
| 5 | 0.76 | 5.0 ± 0.5 | 10.2 ± 0.4 |
| 5 | 1.14 | 4.6 ± 0.2 | 9.3 ± 0.8 |
| 0 | 0.4 | 4.9 ± 0.3 | 9.0 ± 0.5 |
| 0 | 0.8 | 4.0 ± 0.3 | 7.2 ± 0.4 |
| 0 | 1.2 | 3.3 ± 0.1 | 6.1 ± 0.4 |

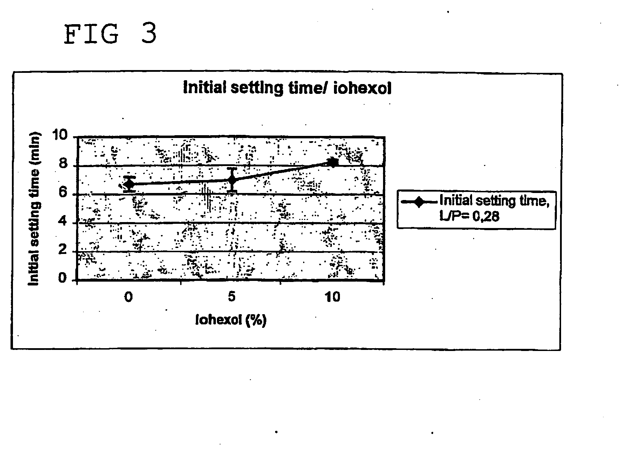

Even for calcium phosphates the setting time is increased if iohexol is added. The more iohexol the longer becomes the setting time, see Figure 3.

### Example 4. Compression test.

The compressive strength is the maximum load a material can withstand without breaking. An Instron 8511.20 mechanical test machine was used for determining the compressive strength.

Paste from mixed powder and distilled water was injected to a PTFE mould. 16 cylindrical samples with a diameter of 4 mm and a height of about 8 mm were made. Calcium phosphate samples were stored in 0.9 % saline solution at a temperature of 37 °C for 14 days before testing, while the samples of mixtures with calcium sulfate were stored in air for 48 hours.

The samples were compressed vertically in the Instron machine at a rate of 1 mm/min until they cracked. The compressive strength C, was calculated as C = F/A, where
F = Force (N) and A = cross section area (m²). The maximum force was used as F.

It was found that the compression strength is not dependent of the amount of accelerator and the compressive strength of a calcium phosphate ceramic decreased when iohexol was added.

### Example 5. Density testing.

The absorption of material in saline solution was investigated for calcium phosphate containing different amounts of iohexol or its dimer iodixanol. Materials containing iohexol or its dimer iodixanol had a lower density after 14 days than materials without the same. This means that these compounds are absorbed in the water and the porosity increases if either of them is added, a higher porosity can promote bone ingrowth.

### Example 6. Scanning Electron Microscope analysis.

For the calcium sulfate based compositions it was impossible to see any difference in the structure if iohexol is added.

The most important benefit with adding iohexol or its dimer iodixanol to calcium sulfates and calcium phosphates is the improved X-ray visibility. For example, adding 10 % iohexol can increase the chance of detecting any leakage when injected to the spine or hip, or at other locations. Complications can be avoided in this fashion. The increased injection time will make the injection easier and give the surgeon more time to work. The increased setting time is no problem because of the possibility to control it with changing the liquid/powder ratio or the amount of accelerator.

## Claims

1. An artificial bone mineral substitute material, which comprises at least one powdered ceramic and at least one water soluble non-ionic X-ray contrast agent, said material comprising calcium sulfate hemihydrate and hydroxylapatite, wherein
the material comprises at least one accelerator, said at least one accelerator being calcium sulfate dihydrate and
hydroxyapatite has a concentration between 20 and 60 weight% of the total weight of the powder components.

2. An artificial bone mineral substitute material as in claim 1, **characterized in that** hydroxyapatite has a concentration of at least 40 weight% of the powder components .

3. An artificial bone mineral substitute material as in claim 1, **characterized in that** said calcium sulfate dihydrate has a concentration between 0.1 and 10 weight% of the total weight of the powder components.

4. An artificial bone mineral substitute material as in claim 1, **characterized in that** said least one water soluble non-ionic X-ray contrast agent is selected from the group comprising iohexol, ioversol, iopamidol, iotrolan, iodixanol, metrizamide, iodecimol, ioglucol, ioglucomide, ioglunide, iogulamide, iomeprol, iopentol, iopromide, iosarcol, iosimide, iotasul, ioxilane, iofratol, and iodecol.

5. A composition as in claim 4, **characterized in that** said at least one water soluble non-ionic X-ray contrast agent is iohexol.

6. A composition as in claim 4 or 5, **characterized in that** said at least one water soluble non-ionic X-ray contrast agent has a concentration between 2 and 20 weight% of the total weight of the powder components.

7. An artificial bone mineral substitute material as in any of claims 1-6, which further comprises at least one osteogenic factor.

8. A composition for an artificial bone mineral substitute material according to any one of claims 1-7, **characterized in that** it further comprises an aqueous liquid.

9. An artificial bone mineral substitute material as in claim 8, wherein the total concentration of calcium sulfate hemihydrate and accelerator is 60 weight% of the total weight of the powder components of the remaining part of the substitute material when ignoring non-ionic X-ray contrast agent, and wherein the ratio between said powder components and said aqueous liquid component (liquid/powder ratio) is between 0.1 and 0.4 ml/g, and wherein said at least one water soluble non-ionic X-ray contrast agent has a concentration between 2 and 20 weight% of the powder components.

10. An artificial bone mineral substitute material as in claim 9, wherein hydroxyapatite has a concentration of 40 weight%, and
calcium sulfate hemihydrate has a concentration between 58.8 and 59.6 weight% of the total weight of the powder components when ignoring non-ionic X-ray contrast agent.

11. A composition as in any one of claims 8-10, **characterized in that** said at least one water soluble non-ionic X-ray contrast agent is dissolved in said aqueous liquid.

12. A composition as in any one of claims 8-10, **characterized in that** said at least one water soluble non-ionic X-ray contrast agent is a dry powder intermixed with said at least one powdered ceramic.

13. A composition as in any one of claims 1-12, which further comprises vitamin E.

14. A composition as in claim 13, **characterized in that** said vitamin E has a concentration between 0.1 and 10 weight% of the total weight of the powder components.

15. A composition as in any of claims 8-14, **characterized in that** said aqueous liquid component is distilled water.

## Patentansprüche

1. Künstliches Knochenmineralien-Ersatzstoffmaterial, welches mindestens ein pulverisiertes keramisches Material und mindestens ein wasserlösliches, nichtionisches Röntgenkontrastmittel umfasst, welches Material Calciumsulfat Halbhydrat und Hydroxylapatit umfasst, wobei
das Material mindestens einen Abbindebeschleuniger umfasst, welcher mindestens eine Abbindebeschleuniger Calciumsulfat-Dihydrat ist, und
das Hydroxylapatit eine Konzentration zwischen 20 und 60 Gew.-% des Gesamtgewichts der Pulverbestandteile aufweist.

2. Künstliches Knochenmineralien-Ersatzstoffmaterial nach Anspruch 1, **dadurch gekennzeichnet, dass** das Hydroxylapatit eine Konzentration von mindestens 40 Gew.-% der Pulverbestandteile aufweist.

3. Künstliches Knochenmineralien-Ersatzstoffmaterial nach Anspruch 1, **dadurch gekennzeichnet, dass** das Calciumsulfat-Dihydrat eine Konzentration zwischen 0,1 und 10 Gew.-% des Gesamtgewichts der Pulverbestandteile aufweist.

4. Künstliches Knochenmineralien-Ersatzstoffmaterial nach Anspruch 1, **dadurch gekennzeichnet, dass** das mindestens eine wasserlösliche nichtionische Röntgenkontrastmittel ausgewählt ist aus der Gruppe umfassend lohexol, loversol, lopamidol, lotrolan, lodixanol, Metrizamid, lodecimol, loglucol, loglucomid, loglunid, logulamid, lomeprol, lopentol, lopromid, losarcol, losimid, lotasul, loxilan, lofratol und lodecol.

5. Zusammensetzung nach Anspruch 4, **dadurch gekennzeichnet, dass** das mindestens eine wasserlösliche nicht-ionische Röntgenkontrastmittel lohexol ist.

6. Zusammensetzung nach Anspruch 4 oder 5, **dadurch gekennzeichnet, dass** das mindestens eine wasserlösliche nicht-ionische Röntgenkontrastmittel eine Konzentration zwischen 2 und 20 Gew.-% des Gesamtgewichts der Pulverbestandteile aufweist.

7. Künstliches Knochenmineralien-Ersatzstoffmaterial nach einem der Ansprüche 1 bis 6, welches ferner mindestens einen osteogenen Faktor enthält.

8. Zusammensetzung für ein künstliches Knochenmineralien-Ersatzstoffmaterial nach einem der Ansprüche 1 bis 7, **dadurch gekennzeichnet, dass** sie ferner eine wässrige Flüssigkeit umfasst.

9. Künstliches Knochenmineralien-Ersatzstoffmaterial nach Anspruch 8, wobei die gesamte Konzentration von Calciumsulfat Halbhydrat und Abbindebeschleuniger unter Nichtbeachtung des nichtionischen Röntgenkontrastmittels 60 Gew.-% des Gesamtgewichts der Pulverbestandteile des übrigen Teils des Ersatzstoffmaterials beträgt, und wobei das Verhältnis zwischen den Pulverbestandteilen und dem wässrigen Flüssigkeitsbestandteil (Verhältnis Flüssigkeit/Pulver) zwischen 0,1 und 0,4 ml/g ist, und wobei das mindestens eine wasserlösliche nichtionische Röntgenkontrastmittel eine Konzentration zwischen 2 und 20 Gew.-% der Pulverbestandteile aufweist.

10. Künstliches Knochenmineralien-Ersatzstoffmaterial nach Anspruch 9, wobei das Hydroxylapatit eine Konzentration von 40 Gew.-% aufweist, und das Calciumsulfat Halbhydrat unter Nichtbeachtung des nichtionischen Röntgenkontrastmittels eine Konzentration zwischen 58,8 und 59,6 Gew.-% des Gesamtgewichts der Pulverbestandteile aufweist.

11. Zusammensetzung nach einem der Ansprüche 8 bis 10, **dadurch gekennzeichnet, dass** das mindestens eine wasserlösliche nichtionische Röntgenkontrastmittel in der wässrigen Flüssigkeit gelöst ist.

12. Zusammensetzung nach einem der Ansprüche 8 bis 10, **dadurch gekennzeichnet, dass** das mindestens eine wasserlösliche nichtionische Röntgenkontrastmittel ein trockenes Pulver vermischt mit dem mindestens einen pulverisierten keramischen Material ist.

13. Zusammensetzung nach einem der Ansprüche 1 bis 12, welche ferner Vitamin E umfasst.

14. Zusammensetzung nach Anspruch 13, **dadurch gekennzeichnet, dass** das Vitamin E eine Konzentration zwischen 0,1 und 10 Gew.-% des Gesamtgewichts der Pulverbestandteile aufweist.

15. Zusammensetzung nach einem der Ansprüche 8 bis 14, **dadurch gekennzeichnet, dass** der wässrige Flüssigkeitsbestandteil destilliertes Wasser ist.

## Revendications

1. Matériau de substitut minéral d'os artificiel, qui comprend au moins une céramique en poudre et au moins un agent de contraste radiographique non-ionique hydrosoluble, ledit matériau comprenant de l'hémihydrate de sulfate de calcium et de l'hydroxyapatite, dans lequel
le matériau comprend au moins un accélérateur, ledit au moins un accélérateur étant le dihydrate de sulfate de calcium, et
l'hydroxyapatite a une concentration comprise entre 20 et 60 % en poids du poids total des constituants en poudre.

2. Matériau de substitut minéral d'os artificiel selon la revendication 1, **caractérisé en ce que** l'hydroxyapatite a une concentration d'au moins 40 % en poids des constituants en poudre.

3. Matériau de substitut minéral d'os artificiel selon la revendication 1, **caractérisé en ce que** ledit dihydrate de sulfate de calcium a une concentration comprise entre 0,1 et 10 % en poids du poids total des constituants en poudre.

4. Matériau de substitut minéral d'os artificiel selon la revendication 1, **caractérisé en ce que** ledit au moins un agent de contraste radiographique non-ionique hydrosoluble est choisi parmi le groupe comprenant l'iohexol, l'ioversol, l'iopamidol, l'iotrolan, l'iodixanol, le métrizamide, l'iodécimol, l'ioglucol, l'ioglucomide, l'ioglunide, l'iogulamide, l'ioméprol, l'iopentol, l'iopromide, l'iosarcol, l'iosimide, l'iotasul, l'ioxilane, l'iofratol et l'iodécol.

5. Composition selon la revendication 4, **caractérisée en ce que** ledit au moins un agent de contraste radiographique non-ionique hydrosoluble est l'iohexol.

6. Composition selon la revendication 4 ou 5, **caractérisée en ce que** ledit au moins un agent de contraste radiographique non-ionique hydrosoluble a une concentration comprise entre 2 et 20 % en poids du poids total des constituants en poudre.

7. Matériau de substitut minéral d'os artificiel selon l'une quelconque des revendications 1 à 6, qui comprend en outre au moins un facteur ostéogénique.

8. Composition pour un matériau de substitut minéral d'os artificiel selon l'une quelconque des revendications 1 à 7, **caractérisée en ce qu'**elle comprend en outre un liquide aqueux.

9. Matériau de substitut minéral d'os artificiel selon la revendication 8, dans lequel la concentration totale de l'hémihydrate de sulfate de calcium et de l'accélérateur est de 60 % en poids du poids total des constituants en poudre de la partie restante du matériau de substitut lorsqu'on ne tient pas compte de l'agent de contraste radiographique non-ionique et dans lequel le rapport entre lesdits constituants en poudre et ledit constituant liquide aqueux (rapport liquide/poudre) est compris entre 0,1 et 0,4 ml/g et dans lequel ledit au moins un agent de contraste radiographique non-ionique hydrosoluble a une concentration comprise entre 2 et 20 % en poids des constituants en poudre.

10. Matériau de substitut minéral d'os artificiel selon la revendication 9, dans lequel l'hydroxyapatite a une concentration de 40 % en poids et l'hémihydrate de sulfate de calcium a une concentration comprise entre 58,8 et 59,6 % en poids du poids total des constituants en poudre lorsqu'on ne tient pas compte de l'agent de contraste radiographique non-ionique.

11. Composition selon l'une quelconque des revendications 8 à 10, **caractérisée en ce que** ledit au moins un agent de contraste radiographique non-ionique hydrosoluble est dissous dans ledit liquide aqueux.

12. Composition selon l'une quelconque des revendications 8 à 10, **caractérisée en ce que** ledit au moins un agent de contraste radiographique non-ionique hydrosoluble est une poudre sèche mélangée avec ladite au moins une céramique en poudre.

13. Composition selon l'une quelconque des revendications 1 à 12, qui comprend en outre de la vitamine E.

14. Composition selon la revendication 13, **caractérisée en ce que** ladite vitamine E a une concentration comprise entre 0,1 et 10 % en poids du poids total des constituants en poudre.

15. Composition selon l'une quelconque des revendications 8 à 14, **caractérisée en ce que** ledit constituant liquide aqueux est l'eau distillée.
